# EUROPEAN PATENT SPECIFICATION

(11) **EP 2 067 440 B1**
(45) Date of publication and mention of the grant of the patent: **23.11.2016**
(21) Application number: 07806349.2
(22) Date of filing: 30.08.2007
(51) Int. Cl.: A61B 5/151, A61B 5/1473, A61B 5/15, A61B 5/157, A61B 5/1459

(54) **DISPOSABLE CARTRIDGE**
EINWEG-KARTUSCHE
CARTOUCHE JETABLE

(30) Priority: 29.09.2006 JP 2006266961
(43) Date of publication of application: 10.06.2009
(73) Proprietor: Terumo Kabushiki Kaisha, Tokyo, 1510072 (JP)
(72) Inventor: NISHIUCHI, Daisuke, Terumo Kabushiki Kaisha, Ashigarakami-gun, Kanagawa 2590151 (JP)
(74) Representative: TBK
(86) International application number: PCT/JP2007/066872
(87) International publication number: WO 2008/041437

(56) References cited:
- WO-A1-2006/028096
- WO-A1-2006/046570
- JP-A- 2004 113 580
- JP-A- 2004 283 568
- US-A- 5 439 473
- US-A1- 2003 050 656
- US-A1- 2004 212 966
- US-A1- 2006 178 686
- US-B1- 6 432 120

## Description

### TECHNICAL FIELD

The present invention relates to a disposable cartridge for use in puncturing the skin of an examinee or measuring a blood component, and more particularly to a disposable cartridge which is prevented from being reinstalled in a device after having been used.

### BACKGROUND ART

In recent years, diabetics are recommended to perform a daily self-managed process for measuring changes in the blood glucose level on their own. In order for a patient to sample its own blood for the measurement of the blood glucose level, it is customary to use a puncture instrument which applies a repulsive force from a resilient member to an axially movable puncture needle to cause the puncture needle to project instantaneously and puncture the skin of the patient for drawing a small amount of blood.

For hygienic reasons, a cartridge including the puncture needle is of the disposable type so that each time a blood glucose level is measured, the cartridge can be detached from the puncture instrument and thrown away and hence will not be used again.

However, the used cartridge may be reinstalled on the puncture instrument by mistake, possibly giving rise to hygienic problems. There is known in the art a puncture cartridge which is capable of preventing a used cartridge from being used again, as disclosed in Japanese Laid-Open

### Patent Publication No. 2004-290390.

According to the disclosed puncture cartridge, a puncture needle is displaceably disposed in a spring member mounted in an outer member. After the puncture needle has punctured the skin, the puncture needle is detached from the spring member. At this time, the spring member shrinks radially inwardly, and the puncture needle that is displaced to the outside of the outer member abuts against the end of the spring member and is locked thereby. Therefore, the puncture needle will not project out of the outer member and puncture the skin, i.e., the puncture cartridge will not be used again.

US patent publication No. 2006/178686 A1 shows a housing, a displacement member displaceable along axial directions of the housing and a tiltable flexible member provided on the housing, wherein the tiltable flexible member has one end supported by the housing and the flexible member is tilted by the displacement member upon displacement thereof, wherein the displacement member has an engaging portion for engaging the one end of the flexible member, and wherein the one end of the flexible member is engaged by the engaging portion.

US patent publication No. 2003/050656 A1 shows a housing, a displacement member displaceable along axial directions of the housing and a tiltable flexible member provided on the housing, wherein the tiltable flexible member has one end supported by the housing.

US patent No. 6432120 B1 shows a housing, a displacement member displaceable along axial directions of the housing and a tiltable flexible member provided on the housing, wherein the tiltable flexible member has one end supported by the housing.

US patent publication No. 2004/212966 A1 shows a housing, a displacement member mounted on the housing and displaceable along axial directions of the housing.

US patent publication No. 5439473 A shows a housing, a displacement member mounted on the housing and displaceable along axial directions of the housing and a tiltable flexible member provided on the housing, wherein the tiltable flexible member has one end supported by the housing and the flexible member is tilted by the displacement member upon displacement thereof.

International patent publication WO 2006/046570 A1 shows a housing, a displacement member mounted on the housing and displaceable along axial directions of the housing.

### DISCLOSURE OF THE INVENTION

According to the background art disclosed in Japanese Laid-Open Patent Publication No. 2004-290390, when the puncture cartridge is assembled, the spring member is temporarily expanded by a guide pipe and a slider with the puncture needle is inserted into the expended spring member. Consequently, the process of assembling the puncture cartridge is highly complex, possibly resulting in an increase in the time required to manufacture the puncture cartridge.

It is a general object of the present invention to provide a disposable cartridge which is of a simple structure, can easily be manufactured, and is reliably prevented from being reinstalled in a device after having been used.

According to the present invention, there is provided a disposable cartridge for being detachably installed in an opening of a device with the features of claim 1.

The disposable cartridge is of a simple structure and can be manufactured with ease, and is reliably prevented from being installed in the device by mistake.

The disposable cartridge may include a puncture needle housed in the housing, and may be installed in a puncture device for puncturing an object with the puncture needle. The disposable cartridge which has been used after the puncture device punctured the object is prevented from being reinstalled in the puncture device. Therefore, hygienic problems due to use of the disposable cartridge are reliably avoided.

The disposable cartridge may include a detection portion disposed in the housing, and may be installed in a measuring device for electrically or optically measuring the detection portion. Consequently, measurement errors caused when the used cartridge is reused are reliably avoided, and the measuring device can produce accurate measured results at all times.

### BRIEF DESCRIPTION OF THE DRAWINGS

FIG. 1 is a perspective view showing a state where a disposable cartridge according to an embodiment of the present invention is detached from a puncture device in which the disposable cartridge is to be installed;
FIG. 2 is a partly sectional perspective view of the puncture device and the disposable cartridge shown in FIG. 1, with a casing and a housing being shown in cross section;
FIG. 3 is an exploded perspective view of the puncture device and the disposable cartridge shown in FIG. 1;
FIG. 4 is a vertical cross-sectional view of the puncture device shown in FIG. 1 which is in a puncturing preparation state;
FIG. 5 is an enlarged cross-sectional view showing the cartridge and nearby parts of the puncture device shown in FIG. 4;
FIG. 6 is a vertical cross-sectional view of the puncture device shown in FIG. 4 which is in a state before the cartridge is installed in the puncture device;
FIG. 7 is an enlarged cross-sectional view showing the cartridge and nearby parts of the puncture device shown in FIG. 6;
FIG. 8 is a vertical cross-sectional view of the puncture device shown in FIG. 6 which is in a state after the housing of the cartridge is installed in the puncture device;
FIG. 9 is a vertical cross-sectional view of the puncture device, showing the manner in which the cartridge shown in FIG. 8 is further inserted into the casing and a slider of the cartridge starts to slide;
FIG. 10 is a vertical cross-sectional view of the puncture device which is in a puncturing state with a puncture needle projecting in response to the pressing of a puncture button of the puncture device shown in FIG. 9;
FIG. 11 is a vertical cross-sectional view of the puncture device, showing the manner in which the puncture needle is pulled back by a return spring after the puncture device shown in FIG. 10 has punctured the skin;
FIG. 12 is a perspective view showing the used cartridge which is taken out and detached from the puncture device shown in FIG. 11;
FIG. 13 is a vertical cross-sectional view of the puncture device shown in FIG. 12;
FIG. 14 is a vertical cross-sectional view showing the manner in which an attempt is made to reinstall the used cartridge in the puncture device shown in FIG. 13;
FIG. 15 is a perspective view of a cartridge according to a first modification;
FIG. 16 is a side elevational view of the cartridge shown in FIG. 15;
FIG. 17 is a side elevational view of the cartridge shown in FIG. 15;
FIG. 18 is a perspective view of a cartridge according to a second modification;
FIG. 19 is a side elevational view of the cartridge shown in FIG. 18; and
FIG. 20 is a side elevational view of the cartridge shown in FIG. 18.

### BEST MODE FOR CARRYING OUT THE INVENTION

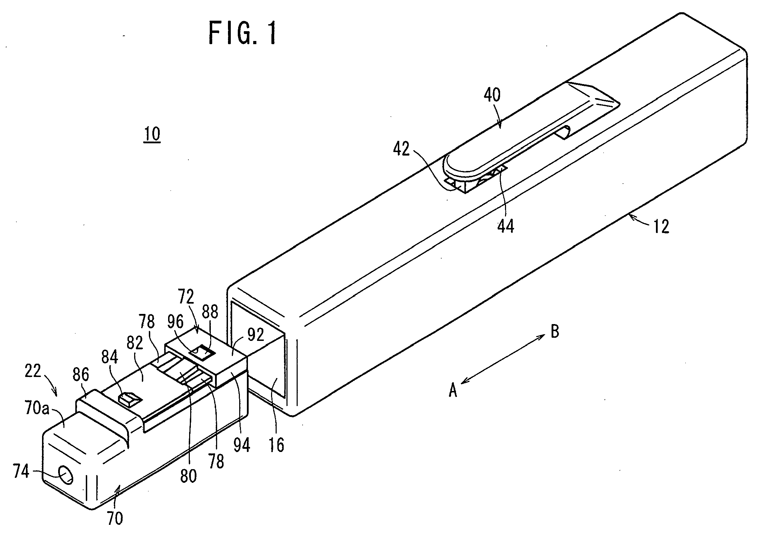

In FIG. 1, the reference character 10 denotes a puncture device, which incorporates a disposable cartridge according to an embodiment of the present invention installed therein, for drawing blood by puncturing the skin of an examinee.

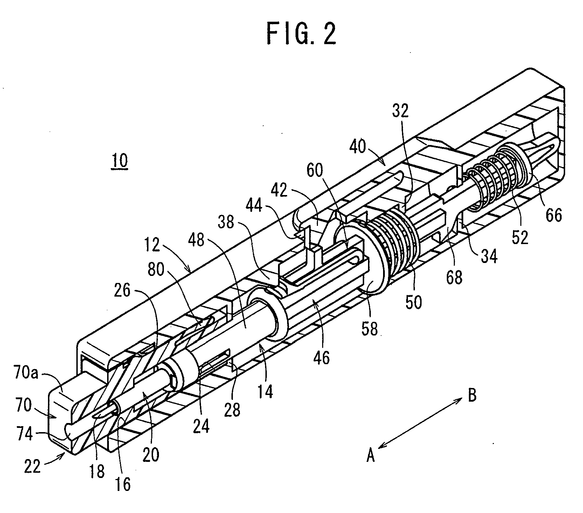

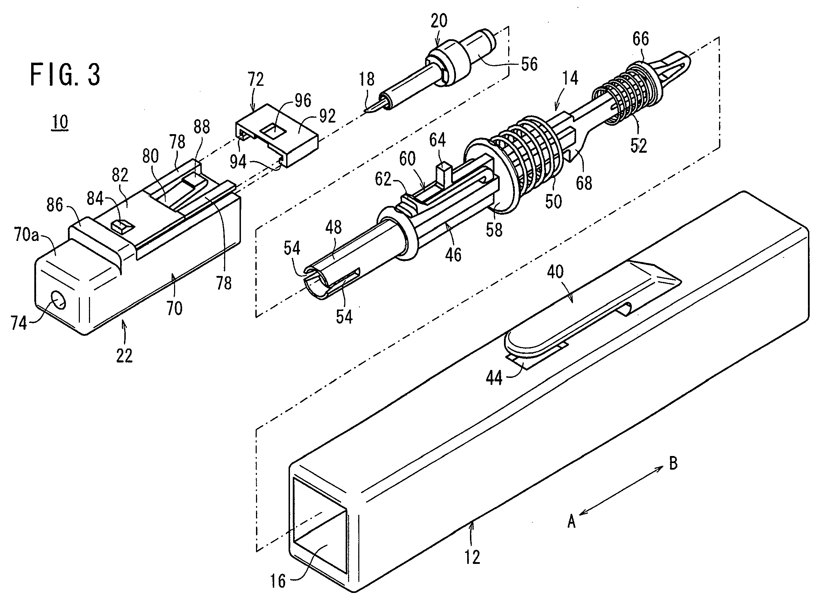

As shown in FIGS. 1 to 4, the puncture device (device) 10 comprises a casing 12 in the form of a bottomed tubular box, a puncture unit 14 displaceably disposed in the casing 12, and a disposable cartridge 22 detachably disposed in an opening 16 of the casing 12 and housing therein a hub 20 having a puncture needle 18.

The casing 12 has a housing hole 24 defined therein which houses the puncture unit 14 therein. The housing hole 24 communicates with the exterior through the opening 16 that is defined in an end of the casing 12. The opening 16 is substantially rectangular in shape, and an inner wall surface of an upper portion which has a puncture button 40 to be described later is greater in height than the housing hole 24. A step 26 is disposed in the boundary region between the opening 16 and the housing hole 24.

The housing hole 24 has therein a stopper wall 28 disposed in an end portion of the casing 12 for engaging the cartridge 22 that is installed in the opening 16, a first unit wall 32 having a first hole 30 defined in a substantially central portion of the casing 12 and through which the puncture unit 14 is inserted, and a second unit wall 34 spaced a predetermined distance from the first unit wall 32 and disposed in another end portion of the casing 12. The second unit wall 34 has a second hole 36 defined centrally therethrough and through which the puncture unit 14 is inserted.

The casing 12 has a locking ledge 38 disposed substantially centrally therein and projecting from the inner wall surface of the housing hole 24 toward the puncture unit 14.

The puncture button 40 is tiltably mounted on a side surface of the other end portion of the casing 12. The puncture button 40 extends substantially parallel to the side surface of the casing 12 and has a pusher 42 projecting from an end portion thereof toward the casing 12. The puncture button 40 has another end portion joined to the side surface of the casing 12. In other words, the end portion of the puncture button 40 which has the pusher 42 is tiltable about the other end portion thereof.

A button hole 44 facing the pusher 42 of the puncture button 40 is defined in the side surface of the casing 12. When the puncture button 40 is tilted about the other end portion of the puncture button 40, the pusher 42 is inserted into the button hole 44.

The puncture unit 14 comprises an axially elongated body 46, a hub gripper 48 disposed on an end portion of the body 46 and releasably holding the hub 20 housed in the cartridge 22, a puncture spring 50 for biasing the body 46 (in the direction indicated by the arrow A) toward the opening 16 of the casing 12, and a return spring 52 for biasing the body 46 in a direction (in the direction indicated by the arrow B) away from the opening 16.

The hub gripper 48, which is of a cylindrical shape with a pair of slits 54 defined therein, is disposed on the end portion of the body 46. The hub gripper 48 is made radially expandable by the slits 54. A reduced-diameter portion 56 of the hub 20 with the puncture needle 18 is inserted in and integrally held by the hub gripper 48.

The body 46 has a flange 58 extending radially outwardly which is disposed on a substantially central portion in the axial directions (the directions indicated by the arrows A, B). A resilient member 60 extends from the flange 58 toward the hub gripper 48 (in the direction indicated by the arrow A). The resilient member 60 lies substantially parallel to the body 46, and tiltable toward and away from the body 46 about the junction between the resilient member 60 and the flange 58.

Specifically, the resilient member 60 is normally biased in a direction away from the body 46. When the end of the resilient member 60 is pressed toward the body 46, the resilient member 60 is tilted toward the body 46 about the junction.

The resilient member 60 has a first protrusion 62 projecting from the distal end thereof in the direction away from the body 46. When the cartridge 22 is housed in the housing hole 24, the first protrusion 62 is engageable with the locking ledge 38. Specifically, when the first protrusion 62 engages the locking ledge 38 of the casing 12, the puncture unit 14 including the body 46 is limited against displacement (in the direction indicated by the arrow A) toward the opening 16 of the casing 12. The resilient member 60 also has a second protrusion 64 projecting from a substantially central portion thereof in the direction away from the body 46, as with the first protrusion 62. The second protrusion 64 is disposed in a position facing the pusher 42 of the puncture button 40. For puncturing the skin, the second protrusion 64 is pushed toward the body 46 by the pusher 42, unlocking the puncture unit 14.

The flange 58 is disposed in the housing hole 24 on one side of the first unit wall 32 nearer to the opening 16 (in the direction indicated by the arrow A). A puncture spring 50 is interposed between the flange 58 and the first unit wall 32 for biasing the flange 58 to move the puncture unit 14 toward the opening 16 (in the direction indicated by the arrow A).

A ring 66 is mounted on the other end portion of the body 46. A return spring 52 is interposed between the ring 66 and the second unit wall 34 for biasing the puncture unit 14 in a direction (the direction indicated by the arrow B) away from the opening 16 of the casing 12.

The body 46 has a locking member 68 disposed between the ring 66 mounted on the other end portion of the body 46 and the flange 58 and projecting toward an inner wall surface of the housing hole 24. The locking member 68 is disposed in the housing hole 24 between the first unit wall 32 and the second unit wall 34. When the body 46 is displaced toward the opening 16 (in the direction indicated by the arrow A) under the resiliency of the puncture spring 50, the locking member 68 abuts against the first unit wall 32, preventing the body 46 from being axially displaced.

The cartridge 22 includes a bottomed tubular housing 70, the hub 20 which is fitted in the housing 70, and a slider (displacement member) 72 displaceable along a side surface of the housing 70.

The housing 70 is of a substantially rectangular cross section, and has an open end which can be mounted in the opening 16 of the casing 12 and a closed other end having a puncture hole 74 through which the puncture needle 18 can be inserted.

The housing 70 has a holding hole 76 for holding part of the hub 20 and a puncture hole 74 communicating with the holding hole 76 and through which the puncture needle 18 of the hub 20 is inserted. The puncture hole 74 is smaller in diameter than the holding hole 76.

The housing 70 has a side surface 70a on which one end portion thereof projects compared to the other end portion thereof. The cartridge 22 including the housing 70 is mounted such that the side surface 70a faces the step 26 of the casing 12.

The side surface 70a has a pair of guide rails 78 with a slider 72, to be described later, being displaceably held thereon, the guide rails 78 being disposed on one end portion of the housing 70, a hook (flexible member) 80 disposed between the guide rails 78 and tiltable with respect to the side surface 70a, a flat land 82 disposed substantially centrally on the housing 70, a slider lock 84 projecting from the land 82, and an engaging wall 86 which is disposed on the end of the land 82 and limits the displacement of the slider 72. The engaging wall 86 is disposed on the other end portion of the housing 70.

The guide rails 78 are spaced from each other and disposed on respective opposite sides of the side surface 70a of the housing 70. The guide rails 78 extend across opposite sides of the land 82 in axial directions (the directions indicated by the arrows A, B) from one end of the housing 70 to an end of the engaging wall 86 disposed on the other end of the housing 70. The guide rails 78 are of a substantially L-shaped cross section which extend vertically in a direction away from the side surface 70a and then extend away from each other. The guide rails 78 are straight substantially parallel to the axial directions (the directions indicated by the arrows A, B) of the housing 70.

The hook 80 is disposed substantially centrally on the side surface 70a between the guide rails 78, and is spaced equal distances from the guide rails 78 in substantially parallel relation thereto. The hook 80 has an end joined to a side of the land 82 and another end inclined upwardly from the joined end. The other end of the hook 80 is thus tiltable about the joined end thereof. The other end of the hook 80 has a tooth 88 projecting away from the side surface 70a of the housing 70.

The land 82 has a flat surface lying substantially flush with the upper surface of the guide rails 78. The slider lock 84, which is of a substantially rectangular cross section, projects to a predetermined height from a substantially central portion of the land 82. The slider lock 84 has a side surface facing toward the guide rails 78 and the hook 80 and serving as a first slanted surface 90 inclined a predetermined angle. The engaging wall 86 extends substantially perpendicularly to the axis of the housing 70, and has a substantially constant height that is slightly greater than the upper surface of the land 82.

The hub 20 is substantially cylindrical in shape. The puncture needle 18 is fixed to one end of the hub 20, and the hub 20 has a reduced-diameter portion 56 on the other end thereof which is inserted in the hub gripper 48 of the puncture unit 14. The hub 20 is thus detachably held by the puncture unit 14.

The slider 72 comprises a plate having a substantially constant thickness. The slider 72 has a flat main body 92 and a pair of guides 94 disposed on respective opposite sides of the main body 92 and engaging the respective guide rails 78 of the housing 70.

The main body 92 has a substantially constant width for covering a portion of the side surface 70a of the housing 70, and has a lock hole (engaging portion) 96 defined substantially centrally therein and providing a substantially rectangular opening. The tooth 88 of the hook 80 and the slider lock 84 are engageable in the lock hole 96. The lock hole 96 includes an inner wall surface facing the housing 70 and serving as a second slanted surface 98 inclined a predetermined angle. When the slider 72 is displaced toward the other end of the housing 70, the second slanted surface 98 faces the first slanted surface 90 of the slider lock 84 and lies substantially parallel to the first slanted surface 90.

The guides 94 are bent substantially perpendicularly closely to the respective sides of the main body 92. The guides 94 are spaced a predetermined distance from the main body 92 in substantially parallel relation thereto. The guides 94 are engaged respectively by the guide rails 78 of the housing 70.

Stated otherwise, the slider 72 is mounted on the housing 70 such that the guides 94 embrace the guide rails 78. The slider 72 is thus held displaceable in the axial directions (the directions indicated by the arrows A, B) of the housing 70 while the guides 94 are being guided by the guide rails 78.

When the slider 72 is displaced toward the other end of the housing 70, the slider 72 passes over the land 82 along the guide rails 78, and is limited against displacement by the engaging wall 86 that projects from the land 82.

If the cartridge 22 has not been in use, i.e., if it has not been installed in the puncture device 10, then the slider 72 is disposed on one end portion of the housing 70 and the tooth 88 of the hook 80 is engaged in the lock hole 96. In other words, the slider 72 is limited by the hook 80 against displacement in the axial directions thereof, and is secured to the one end portion of the housing 70.

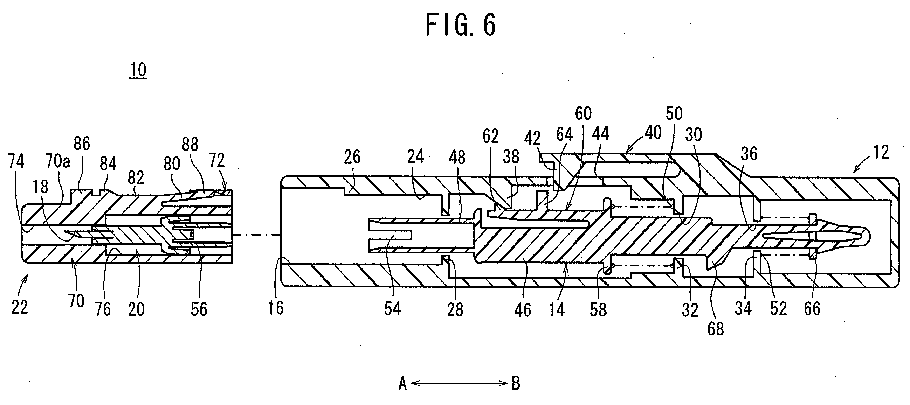

The puncture device 10 for installing therein the cartridge 22 according to the embodiment of the present invention is basically constructed as described above. A process of installing the cartridge 22 in the puncture device 10 will be described below. It is assumed that a state in which the cartridge 22 is not installed in the casing 12 of the puncture device 10 as shown in FIG. 6 is referred to as an initial state.

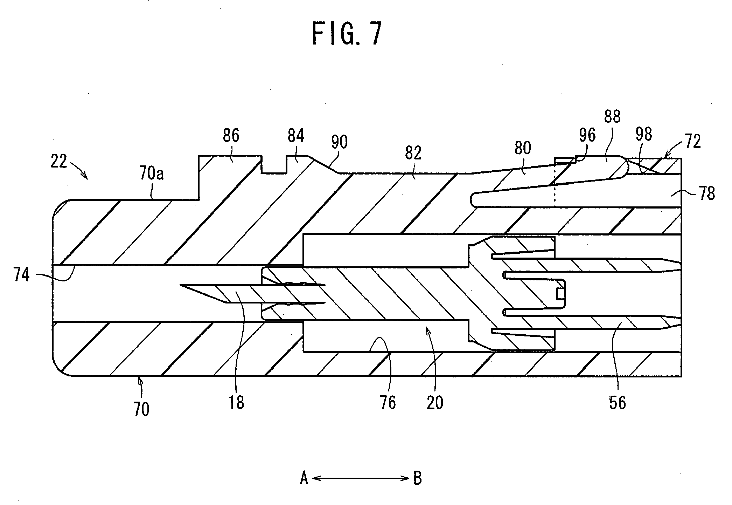

In the initial state, as shown in FIG. 7, the slider 72 of the cartridge 22 is disposed on the one end of the housing 70, and the tooth 88 of the hook 80 is tilted upwardly and engages in the lock hole 96 of the slider 72. Therefore, the slider 72 is limited against displacement by engagement with the hook 80, and held on the one end of the housing 70.

In the puncture device 10, as shown in FIG. 6, the puncture unit 14 is pushed toward the opening 16 (in the direction indicated by the arrow A) under the resiliency of the puncture spring 50, holding the first protrusion 62 of the resilient member 60 in abutment against the slanted surface of the locking ledge 38 of the casing 12.

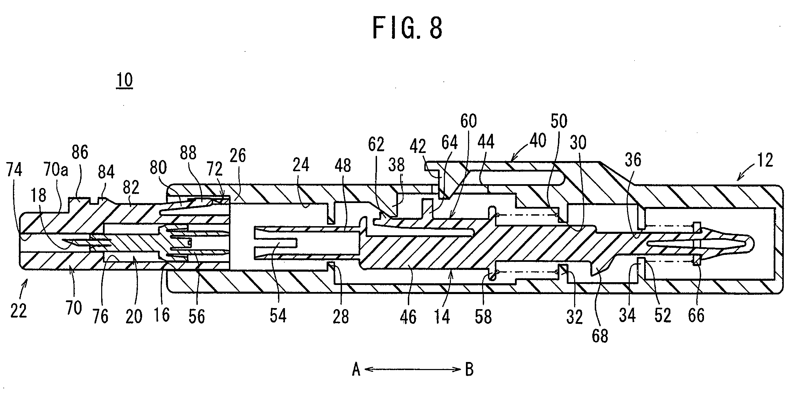

Then, as shown in FIG. 8, the examinee (not shown) grips and inserts the cartridge 22 into the opening 16 of the casing 12 of the puncture device 10 from the open end side of the cartridge 22. The slider 72 engaging and fixed to the side surface 70a of the housing 70 is inserted into the opening 16 until the end of the slider 72 abuts against the step 26.

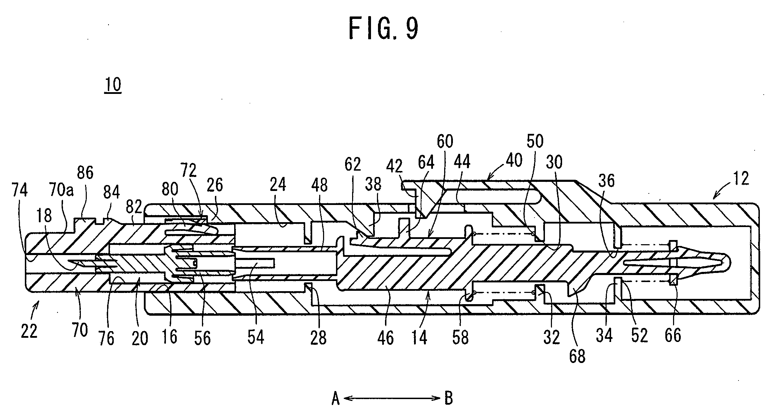

As shown in FIG. 9, while the slider 72 is being limited against displacement by abutment against the step 26, further insertion of the cartridge 22 into the casing 12 causes the tooth 88 of the hook 80 to be guided along the second slanted surface 98 of the lock hole 96 toward the side surface 70a of the housing 70 and disengaged from the lock hole 96. As a result, the slider 72 is released from the restrictive engagement with the tooth 88 of the hook 80, whereupon the housing 70 is displaced toward the puncture unit 14 (in the direction indicated by the arrow B) while the slider 72 is held in engagement with the guide rails 78.

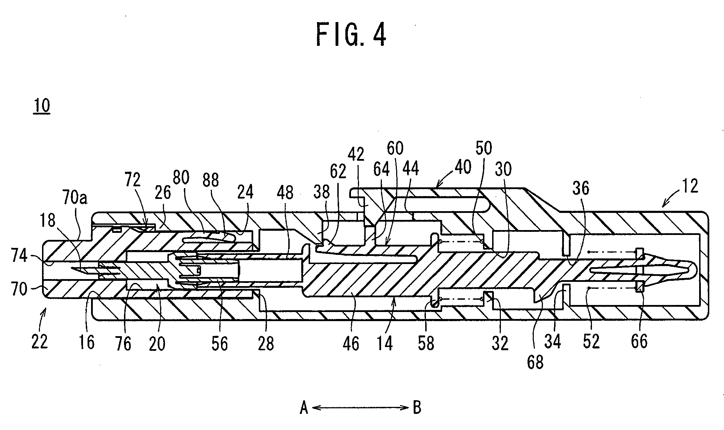

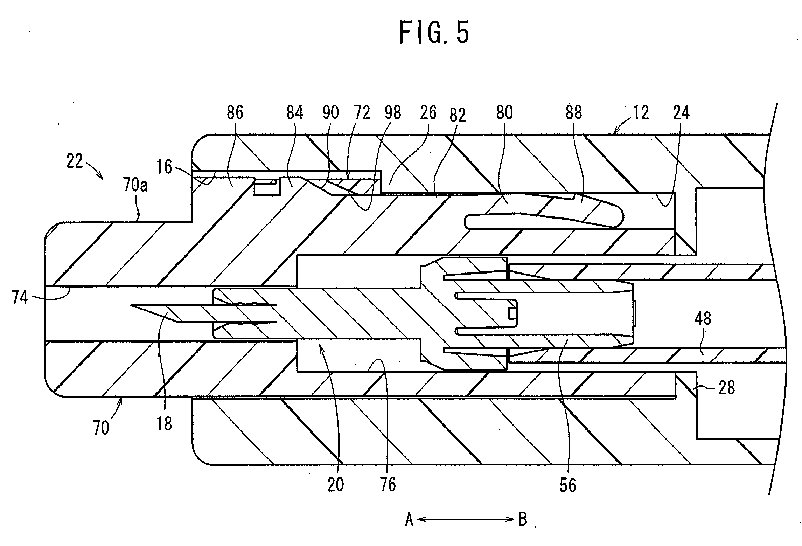

When the cartridge 22 is further pushed into the casing 12, the end of the housing 70 abuts against and is engaged by the stopper wall 28 of the casing 12. The cartridge 22 is now installed in the casing 12 of the puncture device 10, and hence in a puncturing preparation state for a puncturing operation (see FIGS. 4 and 5).

At this time, the reduced-diameter portion 56 of the hub 20 housed in the cartridge 22 is inserted in and held by the hub gripper 48 of the puncture unit 14. The body 46 of the puncture unit 14 is pushed by the hub 20 and displaced in the direction (the direction indicated by the arrow B) away from the opening 16 against the resiliency of the puncture spring 50. The first protrusion 62 of the resilient member 60 rides over the locking ledge 38 and then snaps into engagement with the locking ledge 38 under the resiliency of the puncture spring 50. The second protrusion 64 of the resilient member 60 is disposed so as to face the pusher 42 of the puncture button 40.

At the same time, as the housing 70 is further displaced while the slider 72 is in abutment against the step 26, the land 82 moves into the slider 72 until the slider lock 84 on the land 82 is inserted into the lock hole 96. In the puncturing preparation state, the slider 72 is limited against displacement by engagement with the slider lock 84, and held on the other end of the housing 70 (see FIG. 5).

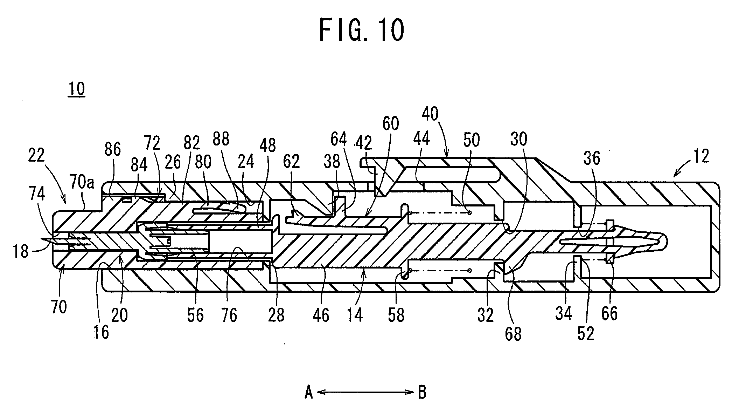

In the puncturing preparation state, for example, the skin of the examinee (not shown) is held against the other end of the cartridge 22, and, as shown in FIG. 10, the examinee presses the puncture button 40 toward the casing 12, thus tilting the puncture button 40. The puncture button 40 pushes the second protrusion 64 of the resilient member 60, tilting the second protrusion 64 toward the body 46 to bring the first protrusion 62 out of engagement with the locking ledge 38. As a result, the body 46 is pushed toward the cartridge 22 (in the direction indicated by the arrow A) under the resiliency of the puncture spring 50. The hub 20 is now displaced with the body 46 integrally.

The puncture needle 18 of the hub 20 projects by a given length through the puncture hole 74 from the other end of the housing 70, and punctures the skin of the examinee, drawing blood from the skin punctured by the puncture needle 18.

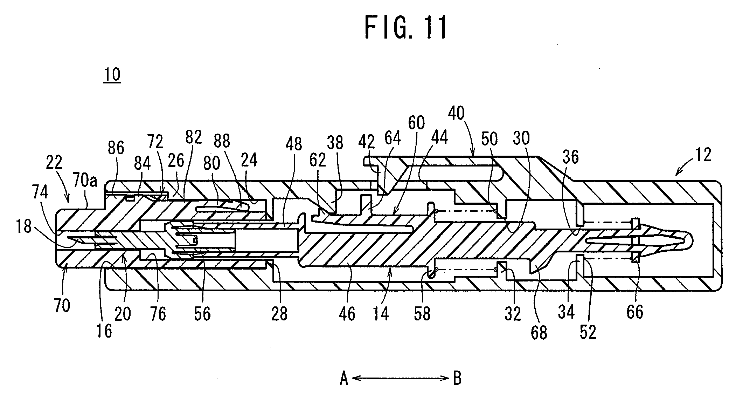

As shown in FIG. 11, in the puncture device 10, the hub 20 having the puncture needle 18 and the body 46 holding the hub 20 are then immediately displaced away from the skin (in the direction indicated by the arrow B) under the resiliency of the return spring 52. The hub 20 and the body 46 are returned to and stopped in a position where the resilient forces of the puncture spring 50 and the return spring 52 are held in equilibrium with each other.

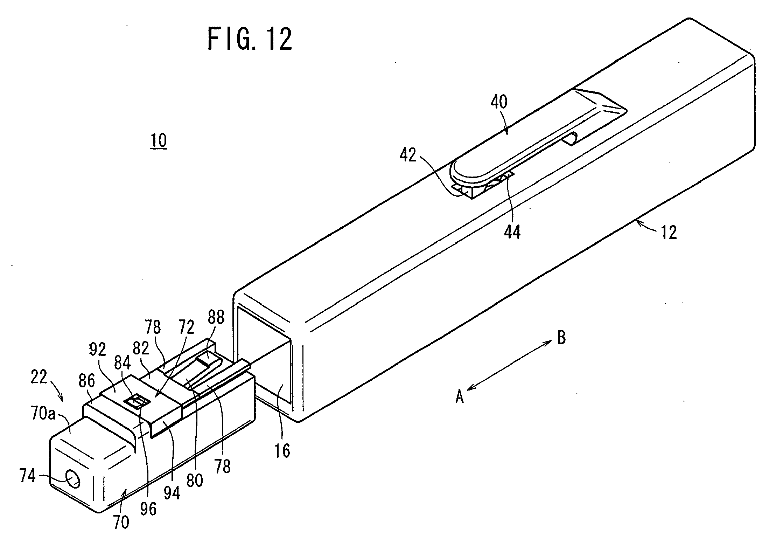

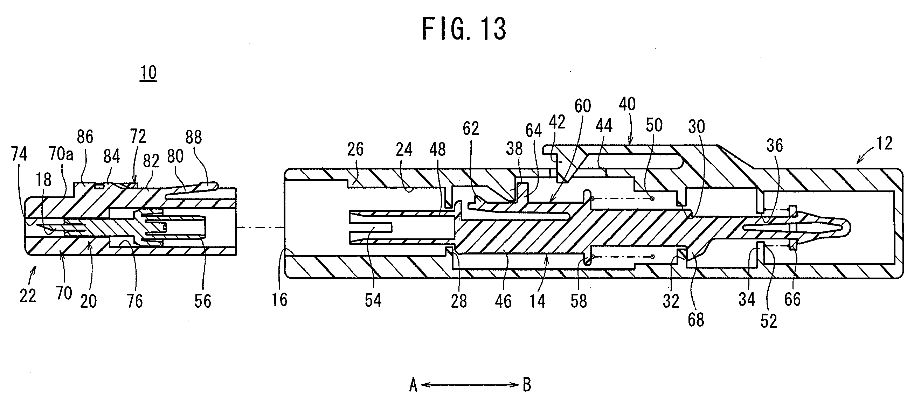

Then, the cartridge 22 is detached from the puncture device 10 after the puncturing process of the puncture needle 18 is completed. As shown in FIGS. 12 and 13, the cartridge 22 is pushed in the direction (the direction indicated by the arrow A) away from the casing 12 by an ejector (not shown), and the examinee grips the cartridge 22 and pulls it in the direction (the direction indicated by the arrow A) away from the casing 12. The locking member 68 of the puncture unit 14 abuts against the first unit wall 32, limiting the puncture unit 14 against displacement, and the hub 20 held by the hub holder 48 is released from the body 46. As a consequence, the hub 20 is taken together with the cartridge 22 out of the puncture device 10.

At this time, since the slider 72 of the cartridge 22 has been displaced along the guide rails 78 after the cartridge 22 was installed in the puncture device 10, the slider 72 is engaged by the slider lock 84 and disposed on the other end portion of the housing 70.

Stated otherwise, before the cartridge 22 is installed in the puncture device 10, the slider 72 is disposed on the one end portion of the housing 70 and held by the tooth 88 of the hook 80. After the cartridge 22 was installed in the puncture device 10, the slider 72 is displaced to the other end portion of the housing 70 and held by the slider lock 84.

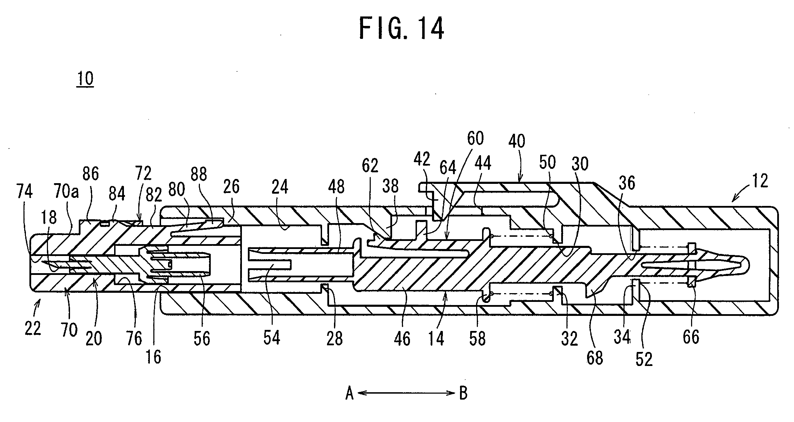

Next, in the case that the used cartridge 22 described above is reinstalled in the puncture device 10 by mistake, operation of the puncture device 10 will be described below with reference to FIG. 14.

As described above, the examinee grips the cartridge 22, which was once installed in the puncture device 10, and inserts the cartridge 22 through the opening 16 into the casing 12. As the slider 72 has already been displaced to the other end of the housing 70 (in the direction indicated by the arrow A) and held by the slider lock 84, the hook 80 is tilted upwardly from the land 82.

Therefore, when the housing 70 is inserted into the opening 16, the other end of the hook 80 with the tooth 88 is brought into abutment against the stop 26, which limits the cartridge 22 including the housing 70 against further displacement. As a result, the used cartridge 22 is reliably prevented from being reinstalled in the puncture device 10 including the casing 12.

In other words, the hook 80 functions a stopper means for limiting the cartridge 22 against displacement, and the slider 72 functions as a switching means for switching the displacement limited state of the cartridge 22 using the hook 80 upon displacement along the guide rails 78.

Stated otherwise, if the cartridge 22 shown in FIG. 6 has not been in use, i.e., if it has not been installed in the puncture device 10, the slider 72 engaged by the tooth 88 of the hook 80 abuts against the step 26, and the hook 80 is pushed downwardly along the first slanted surface 90 of the slider 72. Therefore, the hook 80 is not engaged by the step 26, but is guided into the housing hole 24 of the housing 70.

According to the present embodiment, as described above, the cartridge 22 to be installed in the puncture device 10 includes the displaceable slider 72. If the cartridge 22 has not been used, then the slider 72 is engaged by the tiltable hook 80, and the hook 80 is guided out of abutment against the casing 12 to allow the cartridge 22 to be inserted into the casing 12 of the puncture device 10.

After the cartridge 22 has been installed in the puncture device 10, the slider 72 is brought out of engagement with the hook 80. As the hook 80 will abut against the casing 12, the cartridge 22 is prevented from being inserted into the casing 12 of the puncture device 10.

Accordingly, since the used cartridge 22 is reliably prevented from being reinstalled in the puncture device 10 with a simple structure wherein the slider 72, the hook 80, and the slider lock 84 are provided on the cartridge 22, hygienic problems such as infection due to reuse of the used cartridge 22 are reliably avoided.

Inasmuch as the cartridge 22 can simply be assembled by providing the hook 80 and the slider lock 84 on the side surface 70a of the housing 70 and mounting the slider 72 slidably on the outer surface of the housing 70, the cartridge 22 can be assembled more easily than the puncture cartridges according to the background art.

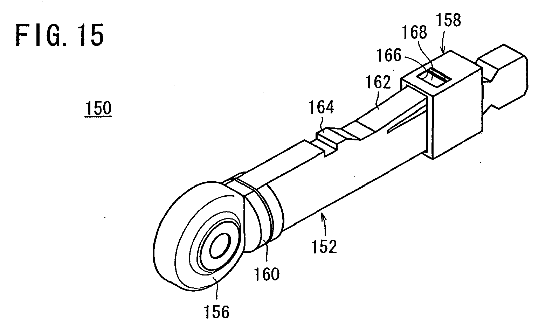

Next, cartridges 150, 200 according to first and second modifications will be described below with reference to FIGS. 15 to 20.

As shown in FIGS. 15 to 17, the cartridge 150 according to the first modification is in the form of a disposable needle hub wherein a puncture needle 154 is insert-molded in a housing 152. The cartridge 150 comprises the housing 152, a cap 156 closing one end of the housing 152, and a slider 158 displaceably mounted on the other end portion of the housing 152.

The cap 156 is of a substantially circular cross section and has a joint 160 on its outer circumferential surface which is joined to the end of the housing 152. When the housing 152 and the cap 156 are angularly displaced relatively to each other, the cap 156 and the housing 152 are separated from each other. Specifically, when the cartridge 150 is used to puncture the skin, the cap 156 is detached from the housing 152 at the joint 160 to expose the puncture needle 154.

A tiltable hook 162 is provided on the other end portion of the housing 152. A slider lock 164 engageable with the slider 158 projects from a substantially central portion of the housing 152.

The slider 158 is of an open tubular shape having a substantially rectangular cross section, and has a lock hole 166 defined in a side surface thereof. A tooth 168 of the hook 162 and the slider lock 164 are engageable in the lock hole 166.

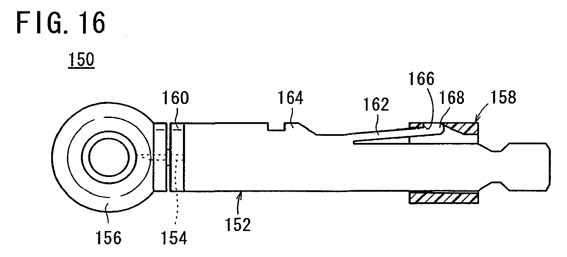

As shown in FIG. 16, before the cartridge 150 is installed in a puncture device (device), not shown, the slider 158 is disposed on the other end portion of the housing 152 with the tooth 168 of the hook 162 engaging in the lock hole 166. The slider 158 is thus held on the other end portion of the housing 152. The slider 158 has a slanted surface for deforming the hook 162.

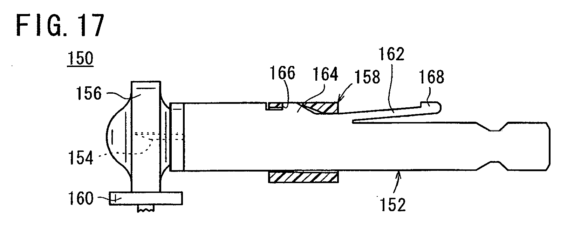

After the cartridge 150 was installed in the puncture device, not shown, the slider 158 has been displaced along the housing 152 toward the one end thereof and engaged and held by the slider lock 164 in the lock hole 166, as shown in FIG. 17. Therefore, the hook 162 is tilted upwardly and abuts against a step in the device, not shown, reliably preventing the used cartridge 150 from being reinstalled.

As shown in FIG. 17, the removed cap 156 is mounted on the used cartridge 150 so as to cover the exposed puncture needle 154. The cap 156 which has been made unnecessary is used to prevent the puncture needle 154 from puncturing the skin by mistake.

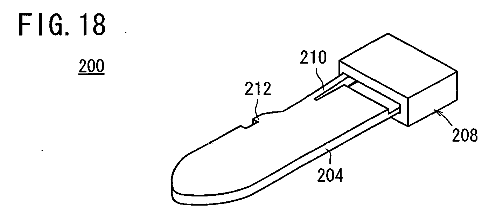

As shown in FIGS. 18 to 20, the cartridge 200 according to the second modification is used as a sensor incorporating a sensing portion (detection portion) 202 for measurement of a blood glucose level or the like based on a current which is produced by a reaction between a small amount of blood and a reagent in the sensing portion 202.

The cartridge 200 comprises a base body (housing) 204, the sensing portion 202 disposed on one end portion of the base body 204 and including an added reagent, electrode terminals 206 electrically connected to the sensing portion 202, and a slider 208 displaceably mounted on the other end portion of the base body 204.

A tiltable hook 210 is mounted on the other end portion of the base body 204. A slider lock 212 engageable with the slider 208 projects from a substantially central portion of the base body 204.

The slider 208 is of an open tubular shape having a substantially rectangular cross section, and has a lock hole 216 defined in a side surface thereof. A tooth 214 of the hook 210 and the slider lock 212 are engageable in the lock hole 216.

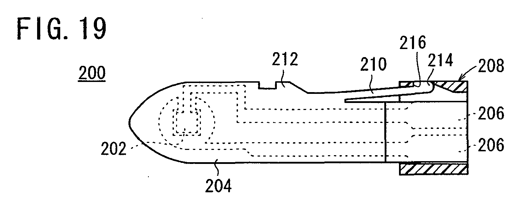

As shown in FIG. 19, before the cartridge 200 is installed in a measuring device (device), not shown, the slider 208 is disposed on the other end portion of the base body 204 with the tooth 214 of the hook 210 engaging in the lock hole 216. The slider 208 is thus held on the other end portion of the base body 204.

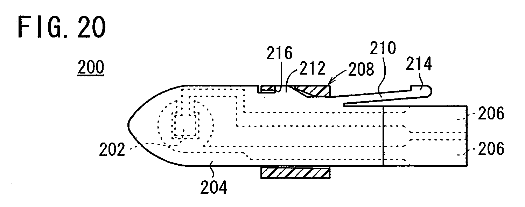

After the cartridge 200 was installed in the measuring device, not shown, the slider 208 has been displaced along the base body 204 toward the one end thereof and engaged and held by the slider lock 212 in the lock hole 216, as shown in FIG. 20.

The used cartridge 200 is thus reliably prevented from being reinstalled in the device. Therefore, inaccurate measured results and measurement failures which would otherwise occur if the used cartridge 200 were installed in the measuring device and reused are reliably avoided. As a result, the measuring device is capable of outputting accurate measured results at all times.

The disposable cartridges according to the embodiments of the present invention are not limited to the above embodiments, but may incorporate various structural details without departing from the scope of the invention.

## Claims

1. A disposable cartridge (22, 150, 200) capable of being detachably installed in an opening (16) of a casing (12) of a puncture device (10) or blood component measuring device, comprising:
a housing (70, 152, 204) insertable into an opening (16) of the puncture device (10) or the measuring device;
a displacement member (72, 158, 208) displaceable along axial directions of the housing (70, 152, 204); and
a flexible member (80, 162, 210) being tiltable with respect to a side surface (70a) of the housing (70, 152, 204) and provided on the housing (70, 152, 204)
wherein the tiltable flexible member (80, 162, 210) has one end supported by the housing (70, 152, 204) and another end capable of facing an opening (16) of the puncture device (10) or the blood component measuring device, and the flexible member (80, 162, 210) is tilted by the displacement member (72, 158, 208) upon displacement thereof, wherein the displacement member (72, 158, 208) has an engaging portion (96, 166, 216) for engaging the other end (88, 168, 214) of the flexible member (80, 162, 210), and wherein before the housing (70, 152, 204) is installed in the puncture device (10) or the blood component measuring device, the other end of the flexible member (80, 162, 210) is engaged by the engaging portion (96, 166, 216);
wherein the displacement member (72, 158, 208) is disposed so as to be capable of abuting against a step (26) disposed in the opening (16) of the puncture device (10) or the blood component measuring device, and when the housing (70, 152, 204) is installed into the puncture device (10) or the blood component measuring device through an opening (16) thereof, the displacement member (72, 158, 208) is displaced along the axial directions with respect to the housing (70, 152, 204) to release the displacement member (72, 158, 208) from the engagement with the flexible member (80, 162, 210); and wherein the flexible member when released from the engagement is disposed as to be capable of abuting against the casing (12) thereby of preventing the cartridge (22, 150, 200) from being reinstalled in a puncture device (10) or blood component measuring device;
**characterized in that**
the displacement member (72, 158, 208) is mounted on the housing (70, 152, 204), and when the housing (70, 152, 204) is installed in a puncture device (10) or blood component measuring device, the flexible member (80, 162, 210) is disengaged from the engaging portion (96, 166, 216) and tilted.

2. A disposable cartridge (22, 150, 200) according to claim 1, wherein the engaging portion (96, 166, 216) comprises a hole (96, 166, 216) in which the other end (88, 168, 214) of the flexible member (80, 162, 210) is insertable.

3. A disposable cartridge (22, 150, 200) according to claim 2, wherein the housing (70, 152, 204) has a lock (84, 164, 212) for engaging in the hole (96, 166, 216) to limit the displacement member (72, 158, 208) against displacement when the housing (70, 152, 204) is installed in a puncture device (10) or blood component measuring device.

4. A disposable cartridge (22, 150) according to claim 1, further comprising a puncture needle (18, 154) housed in the housing (70, 152), the disposable cartridge (22, 150) being installed in a puncture device (10) for puncturing an object with the puncture needle (18, 154).

5. A disposable cartridge (200) according to claim 1, further comprising a detection portion (202) disposed in the housing (204), the disposable cartridge (200) being installed in a blood component measuring device for electrically or optically measuring the detection portion (202).

## Patentansprüche

1. Einwegkartusche (22, 150, 200), die imstande ist, abnehmbar in einer Öffnung (16) einer Hülle (12) einer Einstichvorrichtung (10) oder einer Blutbestandteilmessvorrichtung montiert zu sein, wobei die Einwegkartusche Folgendes aufweist:
ein Gehäuse (70, 152, 204), das in eine Öffnung (16) der Einstichvorrichtung (10) oder der Messvorrichtung einsetzbar ist;
ein Verschiebungsbauteil (72, 158, 208), das entlang axialer Richtungen des Gehäuses (70, 152, 204) verschiebbar ist; und
ein flexibles Bauteil (80, 162, 210), das in Bezug auf eine Seitenfläche (70a) des Gehäuses (70, 152, 204) neigbar ist und an dem Gehäuse (70, 152, 204) vorgesehen ist;
wobei das neigbare flexible Bauteil (80, 162, 210) ein durch das Gehäuse (70, 152, 204) gestütztes Ende und ein anderes Ende hat, das imstande ist, einer Öffnung (16) der Einstichvorrichtung (10) oder der Blutbestandteilmessvorrichtung zugewandt zu sein, und das flexible Bauteil (80, 162, 210) durch das Verschiebungsbauteil (72, 158, 208) bei einer Verschiebung von diesem geneigt wird, wobei das Verschiebungsbauteil (72, 158, 208) einen Eingriffsabschnitt (96, 166, 216) zum Eingreifen mit dem anderen Ende (88, 168, 214) des flexiblen Bauteils (80, 162, 210) hat, und wobei, bevor das Gehäuse (70, 152, 204) in die Einstichvorrichtung (10) oder die Blutbestandteilmessvorrichtung montiert wird, das andere Ende des flexiblen Bauteils (80, 162, 210) durch den Eingriffsabschnitt (96, 166, 216) im Eingriff ist;
wobei das Verschiebungsbauteil (72, 158, 208) angeordnet ist, um imstande zu sein, an einer in der Öffnung (16) der Einstichvorrichtung (10) oder der Blutbestandteilmessvorrichtung angeordneten Stufe (26) anzuliegen, und wenn das Gehäuse (70, 152, 204) in die Einstichvorrichtung (10) oder die Blutbestandteilmessvorrichtung durch eine Öffnung (16) von dieser montiert wird, das Verschiebungsbauteil (72, 158, 208) entlang der axialen Richtungen in Bezug auf das Gehäuse (70, 152, 204) verschoben wird, um das Verschiebungsbauteil (72, 158, 208) von dem Eingriff mit dem flexiblen Bauteil (80, 162, 210) freizugeben; und
wobei das flexible Bauteil (80, 162, 210), wenn es von dem Eingriff freigegeben ist, angeordnet ist, um imstande zu sein, an dem Gehäuse (12) anzuliegen, wodurch die Kartusche (22, 150, 200) daran gehindert wird, wieder in eine Einstichvorrichtung (10) oder eine Blutbestandteilmessvorrichtung montiert zu werden;
**dadurch gekennzeichnet, dass**
das Verschiebungsbauteil (72, 158, 208) an dem Gehäuse (70, 152, 204) angebracht ist, und wenn das Gehäuse (70, 152, 204) in eine Einstichvorrichtung (10) oder eine Blutbestandteilmessvorrichtung montiert ist, das flexible Bauteil (80, 162, 210) von dem Eingriffsabschnitt (96, 166, 216) ausgerückt und geneigt ist.

2. Einwegkartusche (22, 150, 200) gemäß Anspruch 1, wobei der Eingriffsabschnitt (96, 166, 216) ein Loch (96, 166, 216) aufweist, in das das andere Ende (88, 168, 214) des flexiblen Bauteils (80, 162, 210) einsetzbar ist.

3. Einwegkartusche (22, 150, 200) gemäß Anspruch 2, wobei das Gehäuse (70, 152, 204) eine Arretierung (84, 164, 212) zum Eingreifen in das Loch (96, 166, 216) hat, um das Verschiebungsbauteil (72, 158, 208) gegen eine Verschiebung zu begrenzen, wenn das Gehäuse (70, 152, 204) in einer Einstichvorrichtung (10) oder einer Blutbestandteilmessvorrichtung montiert ist.

4. Einwegkartusche (22, 150) gemäß Anspruch 1, des Weiteren mit einer in dem Gehäuse (70, 152) aufgenommenen Einstichnadel (18, 154), wobei die Einwegkartusche (22, 150) in einer Einstichvorrichtung (10) zum Einstechen eines Objekts mit der Einstichnadel (18, 154) montiert ist.

5. Einwegkartusche (200) gemäß Anspruch 1, des Weiteren mit einem in dem Gehäuse (204) angeordneten Erfassungsabschnitt (202), wobei die Einwegkartusche (200) in einer Blutbestandteilmessvorrichtung zum elektrischen oder optischen Messen des Erfassungsabschnitts (202) montiert ist.

## Revendications

1. Cartouche jetable (22, 150, 200) pouvant être installée de manière détachable dans une ouverture (16) d'un boîtier (12) d'un dispositif de piqûre (10) ou dispositif de mesure de composant sanguin, comprenant :
un logement (70, 152, 204) pouvant être inséré dans une ouverture (16) du dispositif de piqûre (10) ou du dispositif de mesure ;
un élément de déplacement (72, 158, 208) pouvant être déplacé le long des directions axiales du logement (70, 152, 204) ; et
un élément flexible (80, 162, 210) pouvant être incliné par rapport à une surface latérale (70a) du logement (70, 152, 204) et prévu sur le logement (70, 152, 204),
dans laquelle l'élément flexible inclinable (80, 162, 210) a une extrémité supportée par le logement (70, 152, 204) et un autre extrémité pouvant faire face à une ouverture (16) du dispositif de piqûre (10) ou du dispositif de mesure de composant sanguin, et l'élément flexible (80, 162, 210) est incliné par l'élément de déplacement (72, 158, 208) suite à son déplacement, dans lequel l'élément de déplacement (72, 158, 208) a une partie de mise en prise (96, 166, 216) pour mettre en prise l'autre extrémité (88, 168, 214) de l'élément flexible (80, 162, 210) et dans laquelle avant que le logement (70, 152, 204) ne soit installé dans le dispositif de piqûre (10) ou le dispositif de mesure de composant sanguin, l'autre extrémité de l'élément flexible (80, 162, 210) est mise en prise par la partie de mise en prise (96, 166, 216) ;
dans laquelle l'élément de déplacement (72, 158, 208) est disposé afin de pouvoir venir en butée contre un échelon (26) disposé dans l'ouverture (16) du dispositif de piqûre (10) ou du dispositif de mesure de composant sanguin et lorsque le logement (70, 152, 204) est installé dans le dispositif de piqûre (10) ou le dispositif de mesure de composant sanguin à travers son ouverture (16), l'élément de déplacement (72, 158, 208) est déplacé le long des directions axiales par rapport au boîtier (70, 152, 204) pour libérer l'élément de déplacement (72, 158, 208) de la mise en prise avec l'élément flexible (80, 162, 210) ; et dans laquelle l'élément flexible, lorsqu'il est libéré de la mise en prise, est disposé afin de pouvoir venir en butée contre le boîtier (12) empêchant ainsi la réinstallation de la cartouche (22, 150, 200) dans un dispositif de piqûre (10) ou un dispositif de mesure de composant sanguin ;
**caractérisée en ce que** :
l'élément de déplacement (72, 158, 208) est monté sur le logement (70, 152, 204) et lorsque le logement (70, 152, 204) est installé dans un dispositif de piqûre (10) ou le dispositif de mesure de composant sanguin, l'élément flexible (80, 162, 210) est dégagé de la partie de mise en prise (96, 166, 216) et incliné.

2. Cartouche jetable (22, 150, 200) selon la revendication 1, dans laquelle la partie de mise en prise (96, 166, 216) comprend un trou (96, 166, 216) dans lequel l'autre extrémité (88, 168, 214) de l'élément flexible (80, 162, 210) peut être insérée.

3. Cartouche jetable (22, 150, 200) selon la revendication 2, dans laquelle le logement (70, 152, 204) a un verrou (84, 164, 212) pour se mettre en prise dans le trou (96, 166, 216) afin de limiter l'élément de déplacement (72, 158, 208) contre le déplacement lorsque le logement (70, 152, 204) est installé dans un dispositif de piqûre (10) ou un dispositif de mesure de composant sanguin.

4. Cartouche jetable (22, 150) selon la revendication 1, comprenant en outre une aiguille de piqûre (18, 154) logée dans le boîtier (70, 152), la cartouche jetable (22, 150) étant installée dans un dispositif de piqûre (10) pour piquer un objet avec l'aiguille de piqûre (18, 154).

5. Cartouche jetable (200) selon la revendication 1, comprenant en outre une partie de détection (202) disposée dans le logement (204), la cartouche jetable (200) étant installé dans un dispositif de mesure de composant sanguin pour mesurer électriquement ou par voie optique la partie de détection (202).
